Europäisches Patentamt

(19) European Patent Office

Office européen des brevets

(11) Publication number: **0 250 374 B1**

## (12) EUROPEAN PATENT SPECIFICATION

(45) Date of publication of patent specification:
02.05.91 Bulletin 91/18

(51) Int. Cl.⁵: **A61K 9/52**, A61K 9/22

(21) Application number: 87830223.1

(22) Date of filing: 15.06.87

(54) Therapeutic system for controlled release of drugs.

(30) Priority: 17.06.86 IT 2080386
29.05.87 IT 2070887

(43) Date of publication of application:
23.12.87 Bulletin 87/52

(45) Publication of the grant of the patent:
02.05.91 Bulletin 91/18

(84) Designated Contracting States:
AT BE CH DE ES FR GB GR IT LI LU NL SE

(56) References cited:
EP-A- 0 077 956
EP-A- 0 177 000
US-A- 3 247 066
CHEMICAL ABSTRACTS, vol. 100, 1984, page
322, abstract no. 180059r, Columbus, Ohio, US;
U. CONTE et al.: "Press-coated, zero-order
drug delivery systems", & FARMACO, ED.
PRAT. 1984, 39(3), 67-75
CHEMICAL ABSTRACTS, vol. 100, 1984, page
324, abstract no. 56791j, Columbus, Ohio, US;
U. CONTE et al.: "Press-coated systems for
drug release control", & POLYM. SCI.
TECHNOL. (PLENUM) 1983, 23(Polym. Med.),
179-86
JOURNAL OF CONTROLLED RELEASE 1,
1985, pages 283-289, Elsevier Science
Publishers, Amsterdam, NL; P. COLOMBO et
al.: "Compressed polymeric mini-matrices for
drug release control"

(73) Proprietor: RECORDATI INDUSTRIA CHIMICA
E FARMACEUTICA S.p.a.
Via M. Civitali, 1
I-20148 Milano (IT)
Proprietor: CONSIGLIO NAZIONALE DELLE
RICERCHE
Via M. Civitale, 1
I-20148 Milano (IT)

(72) Inventor: La Manna, Aldo
Via Lanfranco 3
Pavia (IT)
Inventor: Colombo, Paolo
Via Magenta 12
Pavia (IT)
Inventor: Gazzaniga, Andrea
Via Emilia 27064
Santa Giulietta Pavia (IT)
Inventor: Santus, Giancarlo
Via Zuara 8
Milano (IT)
Inventor: Conte, Ubaldo
Via Treviglio 6
Busto Arsizio (IT)
Inventor: Sangalli, Maria Edvige
Via Beltrami 17
Pavia (IT)

(74) Representative: Righetti, Giuseppe
Bugnion S.p.A. Via Carlo Farini, 81
I-20159 Milano (IT)

## Description

The need for devices able to release active substances with zero-order kinetics is felt in many technological fields. In therapeutic field, the possibility to administer an active ingredient (drug) at controlled rate is of great interest, since this makes it possible to optimize the drug therapeutic effect by maintaining effective and constant blood levels.

Although said aim may be rather easily attained by parenteral administration, such as a slow infusion to hospitalized patient, in the case of oral administration greater difficulties are noticed. Accordingly, several attempts have been made in order to obtain pharmaceutical forms for oral administration with constant drug release kinetics.

It is known that criteria on which the active ingredient controlled release systems are founded, involve different mechanisms, such as :

a) diffusion,

b) chemical erosion,

c) hydration,

d) osmosis, and like.

All said known procedures exhibit disadvantages more or less evident.

In this way, under normal conditions diffusion from matrix systems does not allow for example a zero-order kinetics release, chemical erosion is not a time scaled phenomenon, hydration calls for the use of special polymers, osmosis requires a sophisticated and complicated application technology.

For achieving a controlled release, the matrix type systems, which however show no zero-order release kinetics of the drug, appeared to be more suitable. In order to make the drug administration more reliable and statistically safer, pharmaceutical forms have been realized constituted by several subunits, each of them carrying a portion of the drug dose. Said dosage subunits are generally administered in capsules.

It is further known that under the term zero-order release kinetics, a release rate is meant which remains constant for the entire treatment time, that is mathematically corresponding to the following equation balance: $dC \text{ (plasma)}/dt = dQ_o/dt \cdot 1/(dQ_e/dt \cdot Vd)$, wherein :

$dC \text{ (plasma)}/dt$ means the instant drug plasmatic concentration change,

$dQ_o/dt$ means the instant drug amount change due to the absorption process,

$dQ_e/dt$ is the instant drug amount change due to the elimination process, and

$Vd$ is the drug distribution volume.

Such kinetics may be achieved by using either devices having appropriate geometry, either hydrophobic porous materials or hydrophilic poorly soluble polymers, all tricks being capable of modifying the effective drug diffusion across the protective barrier.

In the US Patent 3,247,066 a dosage form able to delay in a controlled way the drug release is described. The dosage unit was constituted by beads with a uniform drung distribution inside of an hydrocolloidal material and surrounded by a film of synthetic material, the breakdown of which allow the drug inside contained to be delivered in a total and immediate way.

In that application the beads did not present a uniform distribution of all the components, whereas that is a typical characteristic of a matrix structure. Moreover, and this is more important as differentiation element, a fast and immediate drug release take place as soon as the film breaks upon contact with a biological fluid having a well specified pH value. An additional complication is constituted by the composition of the administrat ion dosage form : following US Patent 3,247,066 invention, a mixture of beads with different release pattern have to be used to cover the thereapeutic period between two administrations so that the drug dosage amount is fractionated among a beads population showing different drug release performances.

In J. Contr. Rel. 1, no. 4, 283 (1985), a concise description is given of minimatrices prepared by compression and containing swellable polymers able to be cross-linked on the tablet surface by exposing them to a cross-linking linking agent and/or to UV irradiation.

This technique has several disadvantages correlated to the reactivity of the cross-linking agent, than can interact with the drug substance and promote drug degradation. The cross-linking residual, in addition, constitute surely a toxicity hazard and also the technical production is quite an uneasy tool.

European Patent Application 0077956 describes novel enteric microcapsules containing an active compound as a core material, the coating walls of which consist essentially of ethylcellulose and an enteric polymer material, and optionally a water-swellable polymer material being incorporated into the core material, and a process for the preparation thereof. The microcapsules can easily release the active compound in intestinal tract while protecting the core material sufficiently in stomach.

Object of the present invention was to provide minimatrices containing known active ingredients by employing polymeric materials already known in this field by different type of application, to investigate the polymer type influence on release kinetics, and to modify the active ingredient release from mini-matrix by means of suitable treatments, such as surface cross-linking or film coating, so as to reach kinetics profiles meeting the therepeutical requirements.

For gaining the in advance established ends, and for trying to eliminate, or at least to reduce, all the above mentioned formulations disadvantages of the controlled release administration system, according to known procedures on matrix core, a further polymeric barrier was coated. Said barrier, the permeability of which may be modified by means of suitable interventions when the barrier is under formulation, constitutes a further structural hindrance to the diffusional process of the drug and enables the achievement of a system, new in plan and functionality, overcoming the disadvantages of the traditional reservoir systems. As mentioned above, said reservoir systems have the disadvantage to release the active ingredient with zero-order kinetics only until a content fraction has been released, exhibit a rather long lag time and a drug residue which can not be used for a constant release administration, furthermore possibility exhists of active ingredient dumping.

Object of the present invention is, therefore, a therapeutic system for the controlled rate release of drugs comprising :

a) a deposit (core), obtained by compression, containing besides the active ingredient, preferably 2-90% suitable polymeric materials swellable on contact with water-based fluids, such as gastrointestinal juices or simulated gastrointestinal juices, thus providing a swelling pressure,

b) a polymeric coating deposited on core surface which constitutes a barrier able to control the core swelling by means of an osmotic mechanism,

c) an active ingredient dose which may be distributed in several mini-units containing each a fraction of the therapeutic dose of the drug to be administered, preferably 1-95%.

The polymeric material employed for preparing the core should be selected from swellable polymers, that is : it should be able to increase its volume on contact with water or aqueous fluids, thus generating a force measurable according to known methods (see for example : Int. J. Pharm. Techn. & Prod. Manufact., 5, 1 (1984)).

The polymeric material, that for pharmaceutical applications should be biocompatible and/or biodegradable, is preferably selected from methylcelluloses having different molecular weight, polyvinyl alcohols, acrylic polymers, hydroxypropyl methylcelluloses and generally from any polymer natural or synthetic in nature which is able to swell on contact with water.

Said polymers are for example poly-(N-vinyl-2-pirrolidone), anionic and cationic hydrogels, polyelectrolyte complexes, polyvinyl alcohols with a low acetate residue and cross-linked with glyoxal or formaldehyde or glutaraldehyde or with a dialdehyde acting as cross-linking agent, polyacrylamide cross-linked with methylene-bis-acrylamide, ethylene glycol cross-linked polyacrylic acid, alkylen diamide cross-linked polystyrene sulfonic acid, methyl cellulose cross-linked with a dialdehyde, an agar and carboxymethylcellulose sodium mixture, a water insoluble and water swellable copolymer as described in US patent no. 3 989 586, water swellable N-vinyllactame polymers as disclosed in US patent n. 3 992 562, and others.

In core formulation, further components which are able to provide the end product with suitable characteristics, may be then employed mainly in order to facilitate and/or permit the compression. Said additives selection, their characteristics and the carrier amount, depend fundamentally on solubility of the vehicled active ingredient. If, at contrary, the active ingredient is scarcely soluble, the presence in the core of light soluble additives could be necessary in order to enable the water entrance within the core thus creating a canalized structure, which cause the interaction with polymer and subsequent swelling thereof.

The polymeric coating of dosage mini-matrices should create a continuous and homogeneous barrier. Said barrier may have different composition and structure according to the desired active ingredient release.

Greatly hydrophilic and easily permeable membranes will allow a quick entry of water or aqueous fluids, thus supporting a fast swelling of core polymers and, consequently, the quick release of the active ingredient. If membrane comprises hydrophobic or scantily hydrophilic polymeric materials, and it is scantily water permeable, a remarkable reduction in initial phase of release starting may occur (lag time presence).

Among the polymeric materials which may be employed for the membrane, acrylic polymers, different permeability acrylic copolymers, ethylcellulose, methylcellulose, hydroxypropyl methylcellulose, hydroxypropylcellulose, hydroxyethylcellulose, methylhydroxyethylcellulose and derivatives thereof are included.

In the system of the present invention, as well as in other similar systems, the mini-units working may be synthetized as follows : by dipping in aqueous fluid, the active ingredient release starts, which in said first phase is controlled by the polymeric barrier including the core. Together with the drug release, the water entrance through the membrane causes the core to swell, said swelling being opposed by the membrane barrier. When the pressure exerted by swelling reaches the resistance limit of the membrane, the latter will break allowing the release of the core, which continues to swell and to release drug now no more opposed by the membrane, but by the swelling speed of the polymer (or polymers) constituting the core.

Unlike what occurs in reservoir, said process results in a drug release without the burst phenomenon which is typical of matrices, in a full release of the vehicled amount of the drug, and in residues absence. Therefore the overall release rate may be modified with regard both to core-deposit and barrier composition as well as its thickness.

The invention is now further illustrated with the aid of the following examples which should in no way be construed as limiting the same. Even if in said examples reference was only made to a few known drugs, it is obvious that the present invention may be applied to a great variety of other active ingredients having similar or fully different structure.

## Example 1

For preparing 10,000 release units, the following materials have been employed :

| Materials | |
|---|---|
| Verapamil hydrochloride | 300 g |
| Hydroxypropyl methylcellulose (Methocel K 15 M Colorcon) | 200 g |
| Talc | 500 g |
| Acrylic copolymer (Eudragit RS Rohm Pharma) | 50 g |
| Magnesium stearate | 10 g |
| Acetone / isopropanol 1:1 | 1000 g |

### Core preparation

Hydroxypropyl methylcellulose, talc and active ingredient were mixed together in a suitable mixer. The obtained mixture was wetted with a 5% acrylic polymer solution in acetone/isopropanol 1 : 1, thus providing a homogeneous mass which was forced through a 425 µm grid. The granulate thus obtained was dried in an oven at a temperature of 30-35°C and then lubricated with 0.5% of magnesium stearate.

The mixture was precompressed according to known techniques employing 22 mm diameter flat punches, and operating at a compressive force of about $2 \times 10^8$ Pa (2000 bar).

The compact product forced through a 425 µm grid, was again added with 0.5% of magnesium stearate. For obtaining dosage mini-units, the homogeneous granulate was compressed in a reciprocating tablet machine equipped with normal concave punches (3.5 mm diameter), thus providing cylindrical mini-matrices weighing about 53 mg each (corresponding to an active ingredient content of about 15 mg).

### Mini-units coating

For mini-units coating, a suspension having the following composition was employed :

Materials _____

Low permeability acrylic copolymer

(Eudragit RS - Rohm Pharma)                              3.5 g

Acrylic polymer soluble at pH 1-5

(Eudragit E 100 - Rohm Pharma)                           1.5 g

High permeability acrylic polymer

(Eudragit RL - Rohm Pharma)                              1.0 g

Castor oil                                               0.5 g

FDC no. 4 lacquer                                        0.2 g

Acetone / isopropanol 1:1            q.s. at ml 100

Coating was carried out in a rotating pan according to usual technological procedures, and it was continued until mini-units having a 60 µm coating were obtained.

Release control

Release control was carried out by using Apparatus II of United States Pharmacopoeia USP XXI (paddle), employing simulated gastric fluid (USP) as receiving phase, and operating at 37°C at a rotation speed of 100 rpm. The active ingredient release determination was accomplished spectrophotometrically operating in a continuous flow cell.

Release tests were carried out on :

a) mini-units without control membrane,
b) mini-units with a 60 µm coating membrane.

a) Non-coated mini-units release :

| Time (minutes) | Cumulative drug fraction released % |
|---|---|
| 30 | 30 |
| 60 | 43 |
| 90 | 55 |
| 120 | 66 |

b) 60 µm membrane coated mini-units release :

| Time (minutes) | Cumulative drug fraction released % |
|---|---|
| 30 | 10.0 |
| 60 | 23.7 |
| 90 | 36.5 |
| 120 | 48.5 |
| 180 | 69.0 |

With such a membrane, it was found that the pressure at which said membrane breaks, as measured

5

according to known procedures (I1 Farmaco Ed. Pr. 35, 391 (1980)), was of $2 \times 10^5$Pa (2.0 bar).

## Example 2

Utilizing materials and methods described in Example 1, a core was prepared which was coated according to the same procedure with a coating membrane having a similar composition and a 105 μm thickness. Here below the values of release tests are reported, which were accomplished employing the apparatus described in Example 1.

b) 105 μm membrane coated mini-units release :

| Time (minutes) | Cumulative drug fraction released % |
|---|---|
| 60 | 12.8 |
| 120 | 32.1 |
| 180 | 46.0 |
| 240 | 60.0 |
| 300 | 78.0 |

## Example 3

As described in Example 1, a therapeutic system was prepared utilizing diprophilline as active ingredient. For preparing 10,000 release mini-units the following materials were employed :

| Materials | |
|---|---|
| Diprophylline | 200 g |
| Polyvinyl alcohol (Mowiol 40-88 Hoechst) | 120 g |
| Polyvinyl alcohol (Mowiol 28-99 Hoechst) | 80 g |
| Magnesium stearate | 4 g |
| Ethanol / water 1:1 | 200 g |

## Core preparation

The previously sieved on 300 μm sieves active ingredient, was mixed in a suitable mixer with both the polyvinyl alcohol grades (having different molecular weight and degree of hydrolysis) which were previously ground and forced through a 300 μm sieve. The powder mixture thus obtained was wetted with 200 g of a 1 : 1 ethanol/water mixture, providing a homogeneous mass which was then forced on a 425 μm grid. The granulate thus obtained was dried in an oven at a temperature of 30-35°C, and then lubricated with 0.5% of magnesium stearate. The mixture was precompressed according to known techniques employing 22 mm diameter flat punches, and operating at a compressive force of about $2 \times 10^8$ Pa (2000 bar). The compact product forced through a 425 μm grid, was again added with 0.5% of magnesium stearate. For obtaining dosage mini-units, the homogeneous granulate was compressed in a reciprocating tablet machine equipped with normal concave punches (3.5 mm diameter), thus providing cylindrical mini-matrices weighing about 40 mg each (corresponding to an active ingredient content of about 20 mg).

## Mini-units coating

For mini-units coating, a suspension having proportions and composition as reported in the Example 1 was employed.

Coating was carried out in a rotating pan according to the usual technological procedures, and it was con-

EP 0 250 374 B1

tinued until mini-units having a 70 µm coating membrane were obtained.

Release control

Release was carried out as described in the Example 1. Release tests were carried out on :
a) mini-units without control membrane,
b) mini-units with a 70 µm coating membrane.
a) Non-coated mini-units release :

| Time (minutes) | Cumulative drug fraction released % |
|---|---|
| 15 | 28.8 |
| 30 | 46.2 |
| 60 | 72.3 |
| 90 | 89.6 |
| 120 | 100.0 |

b) 70 µm membrane coated mini-units release :

| Time (minutes) | Cumulative drug fraction released % |
|---|---|
| 30 | 20.0 |
| 60 | 40.9 |
| 90 | 57.1 |
| 120 | 69.5 |
| 180 | 91.4 |
| 240 | 98.0 |

With such a membrane, it was found that pressure at which said membrane breaks, as measured according to known procedures, was of $2{,}3 \times 10^5$ Pa (2.3 bar).

Example 4

Utilizing materials and methods described in Example 1, a core was prepared which was coated according to the same procedure with a coating membrane having a similar composition and a 125 µm thickness. Here below the values of release tests are reported, which were accomplished employing the apparatus described in Example 1.
b) 125 µm membrane coated mini-units release :

7

| Time (minutes) | Cumulative drug fraction released % |
|---|---|
| 30 | 10.5 |
| 60 | 22.0 |
| 120 | 51.4 |
| 180 | 70.9 |
| 240 | 82.8 |
| 300 | 90.4 |
| 360 | 98.2 |

## Example 5

As described in Example 1, a therapeutic system was prepared utilizing dipyridamole as active ingredient. For preparing 10,000 release mini-units the following materials were employed :

| Materials | |
|---|---|
| Dipyridamole | 250 g |
| Polyvinylalcohol (Mowiol 40-88 Hoechst) | 125 g |
| Polyvinylalcohol (Mowiol 28-99 Hoechst) | 75 g |
| Hydroxypropyl methylcellulose (Methocel K15M Colorcon) | 50 g |
| Magnesium stearate | 5g |
| Ethanol / water 1:1 | 200 g |

## Core preparation

The previous sieved on 300 μm sieves active ingredient, was mixed in a suitable mixer with both the polyvinyl alcohol grades (having different molecular weight and degree of hydrolysis) and hydroxypropyl methylcellulose, which were previously ground and forced through a 300 μm sieve. The powder mixture thus obtained was wetted with 200 g of 2 : 1 ethanol/water mixture, providing a homogeneous mass which was then forced on a 425 μm grid. The granulate thus obtained was dried in an oven at a temperature of 30-35°C, and then lubricated with 0.5% of magnesium stearate. The mixture was precompressed according to known techniques employing flat punches having a 22 mm diameter, and operating at a compressive force of about $2 \times 10^8$ Pa (2000 bar). The compact product forced through a 425 μm grid, was again added with 0.5% of magnesium stearate. For obtaining dosage mini-units, the homogeneous granulate was compressed in a reciprocating tablet machine equipped with normal concave punches (3.5 mm diameter), thus providing cylindrical mini-matrices weighing about 50 mg each (corresponding to an active ingredient content of about 25 mg).

## Mini-units coating

For mini-units coating, a suspension having proportions and composition as reported in the Example 1 was employed.

Coating was carried out in a rotating pan according to the usual technological procedures, and it was continued until mini-units having a 35 μm coating membrane were obtained.

Release control

Release was carried out as·described in the Example 1.
Release tests were carried out on :
a) mini-units without control membrane,
b) mini-units with a 35 μm coating membrane.
a) Non-coated mini-units release :

| Time (minutes) | Cumulative drug fraction released % |
|---|---|
| 15 | 16.4 |
| 30 | 28.7 |
| 60 | 38.4 |
| 90 | 48.2 |
| 120 | 58.2 |
| 180 | 72.4 |
| 240 | 79.9 |
| 300 | 80.8 |

b) 35 μm membrane coated mini-units release :

| Time (minutes) | Cumulative drug fraction released |
|---|---|
| 30 | 12.8 |
| 60 | 24.4 |
| 90 | 34.1 |
| 120 | 42.8 |
| 180 | 58.3 |
| 240 | 70.7 |
| 300 | 78.2 |
| 360 | 80.0 |
| 540 | 81.0 |

With such a membrane, it was found that pressure at which said membrane breaks, as measured according to known procedures, was of $2 \times 10^5$ Pa (1.95 bar).

Example 6

Utilizing materials and methods described in Example 1, a core was prepared which was coated according to the same procedure with a coating membrane having a similar composition and a 60 μm thickness. Here below the values of release tests are reported, which were accomplished employing the apparatus described in Example 1.
b) 60 μm membrane coated mini-units release :

9

| Time (Minutes | Cumulative drug fraction released % |
|---|---|
| 30 | 10.4 |
| 60 | 21.8 |
| 90 | 32.3 |
| 120 | 41.5 |
| 180 | 57.4 |
| 240 | 70.9 |
| 300 | 79.1 |
| 360 | 81.3 |

### Example 7

Utilizing materials and methods described in Example 1, a core was prepared which was coated according to the same procedure with a coating membrane having a similar composition and a 75 μm thickness. Here below the values of release tests are reported, which were accomplished employing the apparatus described in Example 1.

b) 75 μm membrane coated mini-units release :

| Time (Minutes) | Cumulative drug fraction released % |
|---|---|
| 30 | 8.9 |
| 60 | 20.2 |
| 90 | 31.1 |
| 120 | 40.9 |
| 180 | 56.9 |
| 240 | 69.1 |
| 300 | 77.9 |

### Example 8

Utilizing materials and methods described in Example 1, a core was prepared which was coated according to the same procedure with a coating membrane having a similar composition and a 110 μm thickness. Here below the values of release tests are reported, which were accomplished employing the apparatus described in Example 1.

b) 110 μm membrane coated mini-units release :

| Time (minutes) | Cumulative drug fraction released % |
|---|---|
| 30 | 3.2 |
| 60 | 10.9 |
| 90 | 19.4 |
| 120 | 28.2 |
| 180 | 44.7 |
| 240 | 58.3 |
| 300 | 69.8 |
| 360 | 75.7 |
| 420 | 78.8 |

## Example 9

Utilizing materials and methods described in Example 1, a core was prepared which was coated according to the same procedure with a coating membrane having a similar composition and a 145 µm thickness. Here below the values of release tests are reported, which were accomplished employing the apparatus described in Example 1.

b) 145 µm membrane coated mini-units release :

| Time (minutes) | Cumulative drug fraction released % |
|---|---|
| 30 | 2.1 |
| 60 | 8.1 |
| 90 | 15.6 |
| 120 | 22.8 |
| 180 | 37.4 |
| 240 | 49.1 |
| 300 | 60.5 |
| 360 | 67.7 |
| 420 | 72.1 |
| 480 | 75.3 |
| 540 | 77.6 |
| 600 | 79.0 |
| 660 | 80.0 |
| 690 | 80.3 |

## Example 10

As described in Example 1, a therapeutic system was prepared utilizing flavoxate hydrochloride as active ingredient.

11

For preparing 10,000 release mini-units the following materials were employed :

| Materials | |
|---|---|
| Flavoxate hydrochloride | 360 g |
| Polyvinylalcohol(Mowiol 40-88 Hoechst) | 120 g |
| Polyvinylalcohol (Mowiol 28-99 Hoechst) | 60 g |
| Hydroxypropyl methylcellulose (Methocel K15M Colorcon) | 60 g |
| Magnesium stearate | 6 g |
| 0.5% Eudragit RS solution in acetone / isopropanol 1:1 | 500 g |

Core preparation

The active ingredient which was previously sieved on 300 µm sieves, was mixed in a suitable mixer with both the polyvinyl alcohols (having different molecular weight and degree of hydrolysis) and hydroxypropyl methylcellulose, which were previously ground and forced through a 300 µm sieve. The powder mixture thus obtained was wetted with 500 g of 0.5% Eudragit RS in acetone/isopropanol 1 : 1, providing a homogeneous mass which was then forced on a 425 µm grid. The granulate thus obtained was dried in an oven at a temperature of 30-35°C, and then lubricated with 0.5% of magnesium stearate. The mixture was precompressed according to known techniques employing flat punches having a 22 mm diameter, and operating at a compressive force of about $2 \times 10^8$ Pa (2000 bar).

The compact product forced through a 425 µm grid, was again added with 0.5% of magnesium stearate. For obtaining dosage mini-units, the homogeneous granulate was compressed in a reciprocating tablet machine equipped with normal concave punches (3.5 mm diameter), thus providing cylindrical mini-matrices weighing 60 mg each (corresponding to an active ingredient content of about 36 mg).

Mini-units coating

For mini-units coating, a suspension having the same proportions and composition reported in the Example 1 was employed.

Coating was carried out in a rotating pan according to the usual technological procedures, and it was continued until mini-units having a 30 µm coating membrane were obtained.

Release control

Release was carried out as described in the Example 1.
Release tests were carried out on :
a) mini-units without control membrane,
b) mini-units with a 30 µm coating membrane.
a) Non-coated mini-units release :

| Time (minutes) | Cumulative drug fraction released % |
|---|---|
| 10 | 10.6 |
| 30 | 18.9 |
| 60 | 30.5 |
| 90 | 42.6 |
| 120 | 54.1 |
| 180 | 76.1 |

b) 30 μm membrane coated mini-units release :

| Time (minutes) | Cumulative drug fraction released % |
|---|---|
| 10 | 0.6 |
| 30 | 2.4 |
| 60 | 6.3 |
| 90 | 12.2 |
| 120 | 19.7 |
| 180 | 36.1 |
| 240 | 51.1 |
| 300 | 73.1 |
| 360 | 89.8 |

With such a membrane, it was found that pressure at which said membrane breaks, as measured according to known procedures, was of $2 \times 10^5$ Pa (1.7 bar).

Example 11

Utilizing materials and methods described in Example 5, a core was prepared which was coated according to the same procedure with a coating membrane having a similar composition and a 50 μm thickness. Here below the values of release tests are reported, which were accomplished employing the apparatus described in Example 3.

b) 50 μm membrane coated mini-units release :

| Time (minutes) | Cumulative drug fraction released % |
|---|---|
| 30 | 0.9 |
| 60 | 2.7 |
| 90 | 5.6 |
| 120 | 9.4 |
| 180 | 19.7 |
| 240 | 31.7 |
| 300 | 44.3 |
| 360 | 57.9 |
| 420 | 73.8 |
| 480 | 88.4 |

## Claims

1. Therapeutic system for the drug controlled release rate comprising :

a) a deposit (core) containing, besides the active ingredient, a polymeric material swellable on contact with water or aqueous fluids thus providing a swelling pressure.

b) a polymeric coating on core surface which constitutes a barrier able to control the core swelling by means of an osmotic mechanism, and

c) an active ingredient dose which may be distributed in several mini-units each containing a fraction of the therapeutic dose of the drug to be administered, characterized in that the polymeric material of a) is selected from the group consisting of hydroxypropyl methylcelluloses, methylcelluloses, polyvinyl alcohols, acrylic copolymers and polymers natural or synthetic in nature able to swell on contact with water, such as poly-(N-vinyl-2-pirrolidone), anionic and cationic hydrogels, polyelectrolyte complexes, polyvinyl alcohols with a low acetate residue level and cross-linked with glyoxal or formaldehyde or glutaraldehyde or with a dialdeyde acting as cross-linking agent, polyacrylamide cross-linked with methylene-bis-acrylamide, ethylene glycol cross-linked polyacrylic acid, alkylendiamine cross-linked polystyrene sulfonic acis, methylcellulose cross-linked with a dialdeyde, a mixture of agar and sodium carboxymethylcellulose, a water insoluble and water swellable copolymer and water swellable N-vinyllactame polymers, and the polymeric coating (b) is obtained from a polymeric material selected from the group consisting of acrylic polymers, acrylic copolymers, ethylcellullose, methylcellulose, hydroxypropyl cellulose, hydroxyethyl cellulose, methylhydroxyethyl cellulose, hydroxypropyl methylcellulose and derivatives thereof.

2. Therapeutic system according to claim 1, characterized in that the polymeric coating (b) deposited on core-deposit is applicable by spraying a polymeric solution and operating in a rotating pan according to known techniques.

3. Therapeutic system according to claim 1, characterized in that the deposit-core is obtainable by compression operating at a pressure of $2 \times 10^8$ Pa (2.000 bar).

4. Therapeutic system according to claims 1 to 3, characterized in that each core-deposit mini-unit contains preferably from 1 to 95% of active ingredient.

5. Therapeutic system according to claims 1 to 4 characterized in that each core-deposit mini-unit contains preferably from 2 to 90% of polymeric material swellable on contact with water.

6. Therapeutic system according to claim 1 to 5, characterized in that a single administration from two or more dosage mini-units may be contained.

7. Therapeutic system according to claims 1 to 6, characterized in that the active ingredient is selected from verapamil, diprophylline, dipyridamole and flavoxate.

## Ansprüche

1. Therapeutisches System zur gesteuerten Freigabe von Arzneimitteln bestehend aus

a) einen Ansatz (Kern), der neben dem Wirkstoff ein polymer Material enthält, das in der Lage ist, bei Kontakt mit Wasser oder mit wässerigen Fluiden anzuschwellen und einen Quellungsdruck damit zu erzeugen;

b) einem auf der Kernoberfläche liegenden Polymerüberzug, welcher eine Sperre darstellt, die die Kernschwellung durch eine osmotische Wirkung steuern kann, und

c) einer Wirkstoffsdosis, die unter mehrere Minieinheiten verteilt werden kann, welche jeweils eine Fraktion der therapeutischen Dosis des zu verabreichenden Arzneimittels enthalten, dadurch gekennzeichnet, daß das unter a) erwähnte polymer Material aus der Gruppe gewählt wird, welche aus den Hydroxypropylmethylcellulosen, den Methylcellulosen, den Polyvinylalkoholen, den Acrylcopolymerisaten und den naturlichen bzw, kunstlichen Polymeren besteht, die imstande sind, bei Kontakt mit Wasser anzuschwellen, wie z. B. Polyvinylpyrrolidon, anionische und kationische Hydrogel, Polyelektrolytenkomplexe, Polyvinylalkoholen mit niedrigem Azetatsruckstand, die mit Glyoxal bzw. Formaldehyd bzw, Glutaraldehyd bzw, mit einen als Vernetzungsmittels diedenden Dialdehyd vernetzt sind, mit Methylen-bis-acrylamid vernetztem Polyacrylamid, mit Athylenglykol vernetzter Polyacrylsäure, mit Alkylendiamin vernetzter Polystyrensulfonsäure, mit einem Dialdehyd vernetzter Methylcellulose, eine Gemish aus Agar-Agar und Natriumcarboxymethylcellulose, ein wasserunlöslischens und wasserquellbares Copolymerisat, sowie wasserquellbaren N-vinyllactam Polymeren, wohin die Polymerumhüllung (b) aus einem polymer Material gewonnen wird, welches aus der aus Acrylpolymeren, Acrylcopolymerisaten, Athylcellulose, methylcellulose, Hydroxypropylcellulose, Hydroxyethylcellulose, Methylhydroxyethylcellulose, Hydroxypropylmethylcellulose und deren Derivaten bestehenden Gruppe gewählt wird.

2. Therapeutisches System nach Anspruch 1, dadurch gekennzeichnet, daß der auf den Kern abgesetzte Polymeruberzug (b) durch Spritzen einer Polymerlösung und inner halb eines drehbaren Beckens in an sich bekannter Weise aufgebracht werden kann.

3. Therapeutisches System nach Anspruch 1, dadurch gekennzeichnet, daß der Kern durch Kompression unter einem Druck von $2^8 \times 10$ Pa (2.000 bar) gewonnen werden kann.

4. Therapeutisches System nach Ansprüchen 1 bis 3, dadurch gekennzeichnet, daß jede Kernminieinheit vorzugsweise von 1 bis 95% Wirkstoff enthält.

5. Therapeutisches System nach den Ansprüchen 1 bis 4, dadurch gekennzeichnet, daß jede Kernminieinheit vorzugsweise von 2 bis 90% an einem wasserquellbaren Polymer enthält.

6. Therapeutisches System nach den Ansprüchen 1 bis 5, dadurch gekennzeichnet, daß in einer Einzelverabreichung zwei oder mehrere Dosierungsminieinheiten enthalten sind.

7. Therapeutisches System nach den Ansprüchen 1 bis 6, dadurch gekennzeichnet, daß der Wirkstoff aus Verapamil, Diprophylline, Dipyridamole und Flavoxate gewalt wird.


## Revendications

1. Système thérapeutique avec délivrance contrôlée de médicaments comprenant :

a) un dépôt (noyau) contenant, en plus de l'ingrédient actif, une matière polymérique qui gonfle au contact de l'eau ou de fluides aqueux ; fournissant ainsi une pression de gonfiement ;

b) un revêtement polymérique sur la surface du noyau lequel constitue une barrière en mesure de contrôler le gonflement du noyau par un mécanisme osmotique ; et

c) une dose d'ingrédient actif qui peut être distribuée en plusieurs mini-unités contenant chacune une fraction de la dose thérapeutique du médicament à administrer, caractérisée en ce que la matière polymérique de "a)" est choisie parmi le groupe comprenant hydroxypropyle méthylcelluloses, méthylcelluloses, alcools polyvinyliques, copolymères acryliques, et polymères naturels ou synthétiques en nature, capables de gonfler en contact de l'eau, tels que poly-(N-vinyle-2-pyrrolidone), hydrogels anioniques et cationiques, complexes polyélectrolytes, alcools polyvinyliques avec un bas niveau résiduel d'acétate et réticules avec du glyoxal ou du formaldéhyde ou du glutaraldéhyde ou avec un dialdéhyde agissant en tant qu'agent de réticulation, polyacrylamide réticulé avec du métylène-bis-acrylamide, acide polyacrylique réticulé avec de l'éthylène-glycol, acide sulfonique de polystyrène réticulé avec de l'alcoylidènediamine, méthylcellulose réticulée avec un dialdéhyde, un mélange d'agar et de carboxyméthylcellulose de sodium, un copolymère insoluble dans l'eau et qui gonfle sous l'influence de l'eau et des polymères de N-vinyllactame qui gonflent sous l'influence de l'eau ; et le revêtement polymérique "b)" est obtenu d'une matière polymérique choisie parmi le groupe comportant des polymères acryliques, des copolymères acryliques, éthylcellulose, méthylcellulose, hydroxy propylcellulose, hydroxyéthylcellulose, méthylhydroxyéthylcellulose, hydroxypropylmé- thylcellulose et dérivés de ces produits.

2. système thérapeutique selon la revendication 1, caractérisé en ce que le revêtement polymérique "b)" déposé sur le noyau-dépôt est applicable par pulvérisation d'une solution polymérique, travaillant dans un récipient rotatif suivant des techniques connus.

3. Système thérapeutique selon la revendication 1, caractérisé en ce que le dépôt-noyau peut être obtenu par compression, travaillant à une pression de $2 \times 10^8$ Pa (2.000 bar).

4. Système thérapeutique selon les revendications 1 à 3, caractérisé en ce que chaque mini-unité de noyau-dépôt contient de préférence 1 à 95% d'ingrédient actif.

5. Système thérapeutique selon les revendications 1 à 4, caractérisé en ce que chaque mini-unité de noyau-dépôt contient de préférence 2 à 90%, de matière polymérique qui gonfle au contact de l'eau.

6. Système thérapeutique selon les revendications 1 à 5, caractérisé en ce que deux ou plus mini-unités de dosage peuvent être contenues dans une administration unique.

7. Système thérapeutique selon les revendications 1 à 6, caractérisé en ce que l'ingrédient active est choisi parmi vérapamil, diprophylline, dipyridamole et flavoxate.